# FASCICULE DE BREVET EUROPEEN

(11) **EP 1 618 104 B1**
(45) Date de publication et mention de la délivrance du brevet: **20.06.2007**
(21) Numéro de dépôt: 04726729.9
(22) Date de dépôt: 09.04.2004
(51) Int. Cl.: C07D 403/04

(54) **PROCEDE DE SYNTHESE DE DERIVES 5-CHLORO-1-ARYL-4-(4,5-DICYANO-1H-IMIDAZOL-2-YL)-3-ALKYL-1H-PYRAZOLE**
PROZESS ZUR SYNTHESE VON 5-CHLORO-1-ARYL-4-(4,5-DICYANO-1H-IMIDAZOL-2-YL)-3-ALKYL-1H-PYRAZOL DERIVATEN
METHOD FOR SYNTHESISING 5-CHLORO-1-ARYL-4-(4, 5-DICYANO-1H-IMIDAZOL-2-YL)-3-ALKYL-1H-PYRAZOLE DERIVATIVES

(30) Priorité: 17.04.2003 FR 0304806
(43) Date de publication de la demande: 25.01.2006
(73) Titulaire: Evultis, 6901 Lugano (CH)
(72) Inventeur: MAZZOLA, Alessandro, CH-6963 Cureggia (CH); SANSO', Giovanni, I-20125 Milan (IT)
(74) Mandataire: Corizzi, Valérie
(86) Numéro de dépôt international: PCT/IB2004/001513
(87) Numéro de publication internationale: WO 2004/092159

(56) Documents cités:
- EP-A- 0 412 849

## Description

La présente invention a pour objet un nouveau procédé de synthèse de dérivés 1-aryl-4-(imidazol-2-yl)-3-alkyl-1H-pyrazole, à partir de dérivés 1-aryl-3-alkyl-1H-pyrazoline-5-one.

Elle concerne plus particulièrement un nouveau procédé de synthèse de dérivés 5-chloro-1-aryl-4-(4,5-dicyano-1H-imidazol-2-yl)-3-alkyl-1H-pyrazole de formule générale (I) : 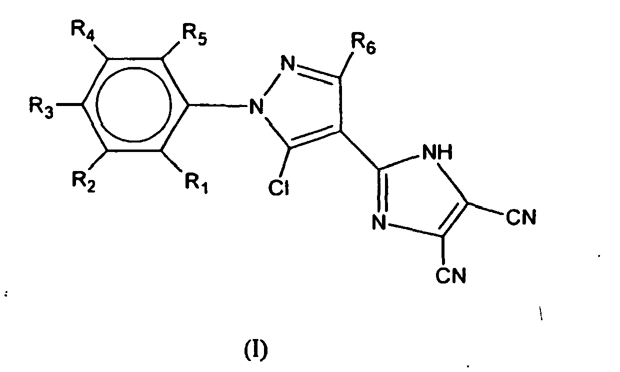 formule dans laquelle :
- R₁ à R₅, identiques ou différents, représentent un groupement choisi parmi :
   * un atome d'hydrogène,
   * un atome d'halogène,
   * un radical répondant à la formule -(X)n-R₇ dans laquelle X représente un groupement choisi parmi l'oxygène, le soufre, un radical sulphinyle et un radical sulphonyle, n est égal à 0 ou à 1 et R₇ représente un radical alkyle linéaire ou ramifié, saturé ou insaturé éventuellement substitué par un ou plusieurs atomes d'halogène identiques ou différents, ce radical alkyle comprenant 1 à 4 atomes de carbone.
- R₆ représente un radical alkyle, linéaire ou ramifié, saturé ou insaturé, comprenant de 1 à 6 atomes de carbone, éventuellement substitués par un ou plusieurs atomes d'halogène, identiques ou différents.

Les composés 1-arylpyrazoles sont connus comme présentant une activité contre un très grand nombre de parasites, dans des domaines aussi larges et variés que l'Agriculture, la Santé Publique et la Médecine Vétérinaire. Les Brevets EP-0 234 119, EP-0 295 117 et US-5,232,940 décrivent une classe d'insecticides et de parasiticides dérivés de N-phénylpyrazoles.

Les composés selon la formule générale (I) ont été décrits dans la Demande Européenne EP-0 412 849 pour leur activité pesticide et insecticide, en particulier pour combattre, dans le domaine de la Médecine Vétérinaire et de l'élevage du bétail, les arthropodes et les helminthes parasites internes ou externes des vertébrés. Ils sont particulièrement utiles pour lutter contre ces parasites sur des vertébrés à sang chaud, les hommes et les animaux tels que les ovins, les bovins, les équidés, les cochons, les chiens et les chats.

Selon la Demande Européenne EP-0 412 849, les composés selon la formule générale (I) sont préparés selon le schéma présenté dans la figure 1, à partir des dérivés 1-aryl-3-alkyl-1H-pyrazoline-5-one, eux-mêmes obtenus de façon classique, à partir de l'arylhydrazine et de l'éthyl 3-alkyl-3-oxopropanoate correspondant.

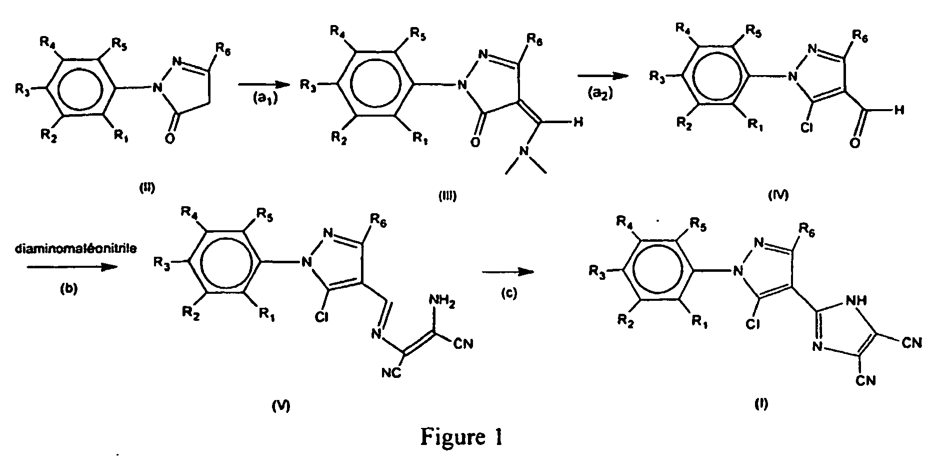

Selon le procédé de l'art antérieur, le dérivé pyrazoline est soumis à l'action du réactif de Vilsmeier pour induire une réaction de formylation et donner accès au 5-chioro-4-carboxaldéhyde correspondant, répondant à la formule générale (IV), via la formation, l'isolation et la purification du dérivé 4-[(diméthylamino)méthylidène] correspondant, qui répond à la formule générale (III).

La transformation du dérivé pyrazoline-5-one (II) en dérivé 5-chloro-4-carboxaldhéhyde (IV) se fait en deux étapes nécessitant une purification intermédiaire et une purification du produit fini, par chromatographie sur colonne de gel de silice.

La transformation de l'aldéhyde (IV) en dérivé selon la formule générale (I) est proposée via l'intermédiaire 4-[(2-amino-1,2 dicyanoéthènylimino)méthyl] répondant à la formule générale (V), obtenu par condensation de l'aldéhyde (IV) avec le diaminomaléonitrile. L'imine (V) conduit au dérivé selon la formule générale (1) par une cyclisation oxydante, qui se fait au moyen du couple N-chlorosuccinimide / nicotinamide ou à défaut en utilisant la 2,3-dichloro-5,6-dicyano-1 ,4-benzoquinone.

La présente Demande a pour objet un nouveau procédé pour la transformation des produits répondant à la formule générale (II) en produits selon la formule générale (I), formules dans lesquelles les variables R₁ à R₆ ont la même définition que ci-dessus, ce procédé ayant un nombre d'étapes réduit par rapport aux procédés de l'art antérieur et requérant le recours à des purifications réduites. En outre, le procédé est doté de meilleurs rendements.

Le procédé de l'invention illustré par la figure 2 ci-dessous est caractérisé en ce que :
(a) dans une première étape, le dérivé pyrazoline-5-one (II) est transformé en dérivé 1-aryl-3-alkyl-4-carboxaldhéhyde-5-chloro-pyrazole de formule (IV) en une étape par traitement de Vilsmeier en présence de POCL₃ et de DMF,
(b) dans une seconde étape l'aldéhyde (IV) est transformé en 1-aryl-3-alkyl-4-[(2-amino-1,2 dicyanoéthènylimino)méthyl]-5-chloro-pyrazole répondant à la formule générale (V) par condensation de l'aldéhyde (IV) avec le diaminomaléonitrile,
(c) dans une troisième étape, l'imine (V) conduit au dérivé selon la formule générale (I) par une cyclisation oxydante, qui se fait par traitement au moyen d'un hypochlorite.

L'invention concerne plus particulièrement les dérivés répondant à la formule (I) dans laquelle n=0.

Avantageusement l'une ou plusieurs des conditions suivantes sont remplies :
- R₁ à R₅, identiques ou différents, représentent un groupement choisi parmi :
   * un atome d'hydrogène,
   * un atome d'halogène,
   * un radical R₇ alkyle linéaire ou ramifié, saturé ou insaturé éventuellement substitué par un ou plusieurs atomes d'halogène identiques ou différents, ce radical alkyle comprenant 1 à 4 atomes de carbone.
- R₆ représente un radical alkyle, linéaire ou ramifié, saturé ou insaturé, comprenant de 1 à 4 atomes de carbone.

Préférentiellement encore, l'une ou plusieurs des conditions suivantes sont remplies :
- R₁ à R₅, identiques ou différents, représentent un groupement choisi parmi :
   * un atome d'hydrogène,
   * un atome de chlore,
   * un radical R₇ alkyle linéaire ou ramifié, saturé ou insaturé éventuellement substitué par un ou plusieurs atomes de fluor, ce radical alkyle comprenant 1 à 4 atomes de carbone.
- R₆ représente un radical choisi parmi le méthyle, l'éthyle, le tertiobutyle, l'isopropyle.

Selon un mode préféré de réalisation de l'invention, celle-ci s'applique à la préparation d'un produit choisi parmi :
le 5-chloro-1-(2,6-dichloro-4-trifluorométhylphényl)-4-(4,5-dicyano-1H-imidazol-2-yl)-3-méthyl-1H-pyrazole,
le 5-chloro-1-(2,6-dichloro-4-trifluorométhylphényl)-4-(4,5-dicyano-1H-imidazol-2-yl)-3-isopropyl-1H-pyrazole,
le 5-chloro- 1-(2,6-dichloro-4-trifluorométhylphényl)-4-(4,5-dicyano-1H-imidazol-2-yl)-3-éthyl-1H-pyrazole,
le 5-chloro-1-(2,6-dichloro-4-trifluorométhylphényl)-4-(4,5-dicyano-1H-imidazol-2-yl)-3-tertiobutyl-1H-pyrazole.

L'art antérieur enseigne l'existence de quelques procédés généraux pour l'incorporation d'un groupe 4,5-dicyano-1H-imidazol-2-yl dans une structure aliphatique ou hétérocyclique à partir de diaminomaléonitrile. Il est possible, selon R. W. Begland, J. Org. Chem. 39 (16), 2341, 1974 d'utiliser des orthoesters ou des orthoamides, de préparer intermédiairement des dérivés de monocondensation du diaminomaléonitrile avec des chlorures ou anhydrides d'acides, ou de passer par la formation d'une mono base de Schiff suivie d'une cyclisation oxydante.

Le procédé de l'invention propose des conditions réactionnelles qui permettent d'éviter l'isolation et la purification de l'intermédiaire (III). Ces conditions réactionnelles comportent une méthode de cyclisation oxydante plus propre et plus facilement adaptable à l'échelle industrielle pour la dernière étape.

Selon une variante préférée du procédé de l'invention, il est possible de transformer les pyrazolones répondant à la formule générale (II) vers les dérivés selon la formule générale (I) en isolant et purifiant uniquement l'intermédiaire aldéhyde (IV), soit en uniquement deux étapes, dans des conditions particulièrement conforme à l'usage industriel et avec des rendements extrêmement compétitifs.

Les améliorations et modifications faisant l'objet de la présente invention, schématisées dans la figure 2, sont détaillées comme suit :

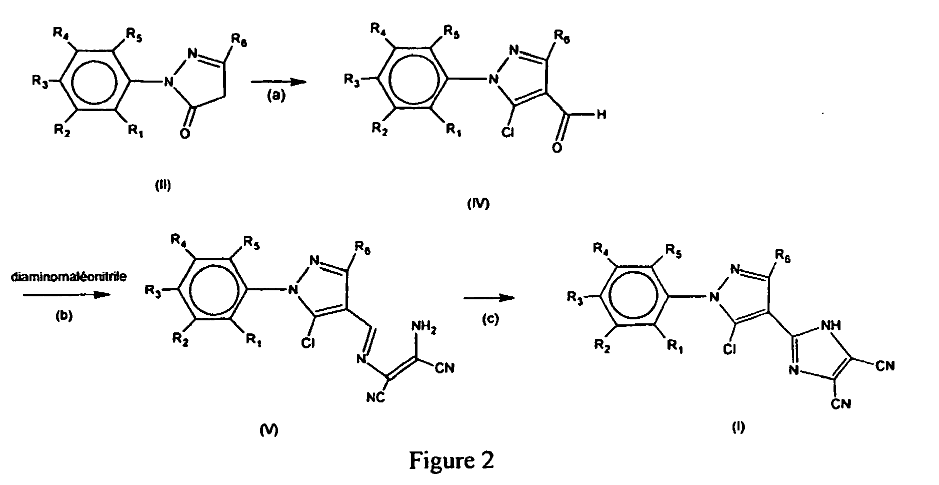

Les étapes (a₁) et (a₂) selon la figure 1, sont remplacées avantageusement par une seule étape (a) telle qu'illustrée sur la figure 2.

Les réactifs de Vilsmeier, habituellement employés pour l'introduction d'une fonction carboxaldéhyde sur un motif hétérocyclique, sont généralement préparés via la réaction d'une N,N-dialkylamide, telle que la N,N-diméthylformamide, avec un réactif de condensation et (ou) de déshydratation. Les réactifs préférés sont par exemple le chlorure d'oxalyle, le phosgène, le trichlorure de phosphoryle employés dans des solvants du type non protique et en particulier chlorés.

Selon le procédé de l'invention, l'étape (a) est mise en oeuvre par traitement du composé de formule (II) dans le DMF en présence de 20 à 40 équivalents molaires de POCl₃, préférentiellement 25 à 35 équivalents molaires de POCl₃, encore plus préférentiellement 30 équivalents molaires de POCl₃.

Cette réaction se fait avantageusement en présence d'un ratio (II)/DMF allant de 1 à 2, encore plus avantageusement de 1 à 1,5 et préférentiellement de 1 à 1,2.

Ces conditions réactionnelles permettent :
- d'accéder au produit (IV) sans isolement et purification intermédiaire du produit de formule (III) ;
- de limiter le volume des rejets et donc de réduire les contraintes environnementales ;
- d'obtenir le produit (IV) avec un rendement de 85% après purification par chromatographie sur colonne de silice, quand le procédé de l'art antérieur ne donnait que 50% de rendement sur ces étapes et nécessitait d'avoir recours à environ 250 équivalents molaires de POCl₃ (EP-0 412 489).

L'étape (b) selon la figure 2 est améliorée par rapport à l'étape (b) selon la figure 1, telle que mentionnée dans la Demande Européenne EP-0 412 849. La formation de l'imine selon la formule générale (V) est habituellement conduite en milieu solvant tels que solvants aromatiques et plus précisément le benzène ou le toluène, en milieu solvants chlorés ou alcools aliphatiques tels que méthanol ou éthanol, à une température comprise entre 0 et 70°C.

Selon le procédé de l'invention, la réaction est conduite de préférence en milieu méthanolique avec une catalyse acide. Parmi les acides susceptibles d'être utilisés on peut citer : l'acide acétique, l'acide paratoluènesulphonique, l'acide trifluoroacétique, l'acide sulfurique, l'acide méthanesulphonique.

Selon un mode préféré de réalisation de la présente invention, la réaction est catalysée par l'acide trifluoroacétique et permet d'obtenir un rendement quasiment quantitatif à l'étape (b).

L'étape (c) telle qu'illustrée par la figure 2 est réalisée par traitement du composé répondant à la formule (V) par un hypochlorite, tel qu'un hypochlorite de métal alcalin ou alcalino-terreux ou un hypochlorite d'alkyle. On peut citer par exemple parmi les hypochlorites utilisables dans le procédé de l'invention : l'hypochlorite de ter-butyle, l'hypochlorite de sodium, l'hypochlorite de calcium, l'hypochlorite de lithium. La réaction est généralement mise en oeuvre dans un solvant aliphatique hydroxylé, à une température comprise entre -5°C et 25°C, préférentiellement entre 0°C et 5°C.

Avantageusement, on utilise 1 à 5 équivalents molaires d'hypochlorite par rapport au produit (V), encore plus préférentiellement 2 à 3 équivalents molaires. Parmi les solvants utilisables pour la mise en oeuvre de cette étape, on peut citer : le méthanol, l'éthanol, le propanol.

Selon les procédés de l'art antérieur, la cyclisation oxydante de l'imine de formule (V) se faisait (EP-0 412 849) par traitement avec le couple N-chlorosuccinimide / nicotinamide, la nicotinamide étant un potentiateur de l'activité oxydante de la NCS (Cf. MORIYA O. et Coll., Synthésis, (1984), 12, p. 1057-58).

Par les mêmes auteurs, on connaît la faible réactivité de la 2,3-dichloro-5,6-dicyano-1,4-benzoquinone et du diiminosuccinonitrile, réactifs oxydants nécessitant des durées de réaction au reflux dans l'acétonitrile pouvant aller de 17 heures à 4 jours pour la cyclisation oxydante de ces mêmes bases de Schiff. Dans les conditions préconisées par O. Moriya, la transformation d'un produit (V) en produit selon la formule générale (1) est réalisée avec un rendement limité à 56% après chromatographie sur gel de silice. L'utilisation d'un tel couple nécessite en effet, une purification délicate, le produit brut résultant contenant trois hétérocycles azotés de polarités voisines.

Selon la même Demande Européenne EP-0 412 849, la cyclisation peut également être conduit avec la DDQ ou 2,3-dichloro-5,6-dicyano-1,4-benzoquinone, réactif dont l'utilisation industrielle est limitée. D'autre part, le sous-produit formé, soit le 1,4-dihydroxybenzène correspondant, n'est pas exempt de toxicité et nécessite un traitement significatif des rejets aqueux. Il est connu, d'autre part, que ce réactif n'offre pas en générale un taux de transformation appréciable, qu'il induit une coloration marquée du produit résultant, qu'il nécessite une durée de réaction longue et le choix d'une température de réaction élevée, par exemple au reflux de l'acétonitrile. Ces observations et caractéristiques se retrouvent en particulier dans le Brevet US-5,380,865, pour une réaction similaire utilisant cet oxydant et la 1-amino-2-(2,6-dichloro-4-trifluorométhylbenzylidèneamino)-1,2-dicyanoéthylène et conduisant au 2-aryl-4,5-dicyanoimidazole correspondant sous la forme d'un solide brun avec un rendement de 42,5%.

L'art antérieur cite de nombreux réactifs autres que ceux ci-dessus mentionnés qui ont été proposés pour la cyclisation oxydante de la base de Schiff correspondante (produit (V)). Le Brevet US-5,380,865 propose de façon générale pour l'accès aux dérivés 2-aryl-4,5-dicyanoimidazole l'emploi des associations iode-acétate de sodium ou brome-acétate de sodium dans un solvant inerte tel que le dichlorométhane ou le diméthylformamide. L'emploi du tétraacétate de plomb est préconisé pour la même transformation par Eicher T. et Coll., Tetrahedron Lett., (1980), 21, 3751-54 et dans le Brevet US-4,220,466 pour l'accès respectivement au 2-isopropyl-4,5-dicyanoimidazole et au 2-ter.butyl-4,5-dicyanoimidazole.

L'utilisation de diiminosuccinonitrile est mentionnée pour la préparation de 2-ter.butyl-4,5-dicyanoimidazole, en milieu acétonitrile à reflux, avec un rendement de 57% par R. W. Begland et Coll., Chem. (1974), 39, p. 2341-2350.

Il a été rapporté par J. P. Ferris, J. Org. Chem., 52(12), 2355-61, (1987) que l'hypochlorite de ter.butyle pouvait, en milieu acétate d'éthyle et dans des conditions relativement douces, contribuer à la conversion d'un dérivé ribose acyclique incorporant un reste iminoaminomaléonitrile vers un dérivé 2-substitué-4,5-dicyanoimidazole, avec un rendement de 66%. L'emploi du N-bromosuccinimide en milieu acétate d'éthyle et à une température modérée a été clairement mentionné pour une transformation analogue et un même rendement dans le même document.

Toutefois, contrairement à l'enseignement de ce dernier document, l'emploi de l'hypochlorite pour réaliser l'étape (c) du procédé de l'invention donne des résultats très supérieurs en termes de rendement par rapport à l'emploi de N-halogénosuccinimide (exemple comparatif 5-2). Dans le procédé de l'invention, on obtient un taux de conversion des composés de formule (V) en composés de formule (I) très supérieurs à ce que laissait espérer la publication précitée.

L'emploi d'un hypochlorite pour cette étape présente de nombreux avantages. Les hypochlorites sont des produits de plus large diffusion industrielle par rapport à la plupart des réactifs mentionnés dans l'art antérieur. Ces hypochlorites sont de plus d'un coût nettement plus attractif que les réactifs de l'art antérieur.

Le réactif particulièrement préféré selon la présente invention pour l'étape (c) / figure 2 est l'hypochlorite de sodium. On choisit plus précisément l'utilisation d'un hypochlorite de sodium présentant une teneur en chlore actif voisine de 150g/litre (tel celui commercialisé par Solvay Electrolyse) ou l'utilisation d'un produit garanti avec un teneur en chlore actif de 315g/litre (tel celui commercialisé par Atofina, division Chlorochimie). A la différence des réactifs précédemment cités dans l'art antérieur pour cette transformation, les hypochlorites réagissent dans des conditions de température plus douces et avec une cinétique plus rapide. On peut citer pour mémoire les deux exemples précédemment évoqués et utilisant la 2,3-dichloro-5,6-dicyanobenzoquinone dans l'acétonitrile pour la cyclisation oxydante, dans la Demande EP-0 412 849 comme dans la publication Synthesis, (1984), 12, p. 1057-58, avec une transformation de l'imine au reflux et respectivement en 12 et 17 heures au minimum.

L'utilisation de l'hypochlorite de sodium répondant aux caractéristiques mentionnées plus haut permet de limiter la durée de la transformation à 0.5 hr et, par un choix judicieux du volume de solvant, notamment de méthanol, de favoriser quasi exclusivement l'élimination d'acide chlorhydrique à partir de la chloramine intermédiaire au dépens de la régénération de l'aldéhyde d'origine.

Selon cette variante de l'invention, le produit de formule générale (V) est traité :
- dans le méthanol,
- à une concentration molaire de (V) allant de 0,005 M à 0,1 M, avantageusement de 0,01 M à 0,08M, encore plus préférentiellement de 0,02 M à 0,06 M,
- par un hypochlorite en qualité allant de 1 à 5 équivalents molaires, préférentiellement de 2 à 3 équivalents molaires par rapport au produit (V), cet hypochlorite étant en solution aqueuse de concentration allant de 1 à 5 M, préférentiellement de 2 à 5 M.

D'autre part, contrairement aux réactifs selon l'art antérieur, les hypochlorites, dans les conditions de mise en oeuvre employées dans le procédé selon la présente invention, n'engendrent pas la formation de sous-produits aromatiques et/ou hétérocycliques dont l'élimination est laborieuse et coûteuse.

Selon une variante particulièrement attractive du procédé selon la présente invention, on transforme les 1-aryl-3-alkyl-1H-pyrazoline-5-one selon la formule générale (II) en un produit répondant à la formule générale (I) suivant un enchaînement réactionnel limité à deux étapes, le seul intermédiaire isolé et purifié étant l'aldéhyde répondant à la formule générale (IV).

Le schéma réactionnel est représenté sur la figure 3 :

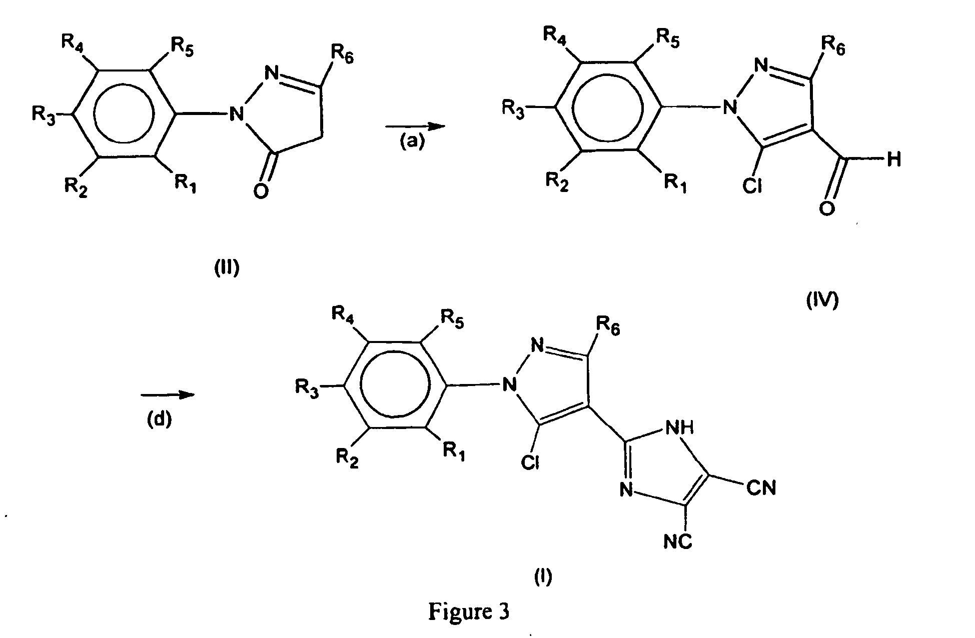

Selon ce schéma, l'étape (a) est identique à celle présentée sur la figure 2 et détaillée plus haut. L'étape (d) selon le même schéma illustré par la figure 3 ne comporte pas d'étape de purification de l'imine intermédiaire répondant à la formule générale (V) et vient donc en lieu et place des étapes (b) et (c) selon la figure 2. Cette réduction du nombre d'étapes est permise par la définition et le choix d'un système réactionnel monophasique à même de favoriser dans la continuité la formation de l'imine et la cyclisation oxydante, l'agent oxydant choisi de préférence étant l'hypochlorite de sodium.

Selon ce schéma :
(a) dans une première étape, le dérivé pyrazoline-5-one (II) est transformé en dérivé 1-aryl-3-alkyl-4-carboxaldhéhyde-5-chloro-pyrazole de formule (IV) en une étape par traitement de Vilsmeier en présence de POCL₃ et de DMF,
(a) dans une seconde étape, par traitement successif du composé de formule (IV) par le diaminomaléonitrile puis par un hypochlorite.

Cette variante permet d'obtenir des rendements allant jusqu'à plus de 85% sur l'étape (d).

Une des variantes particulièrement préférée du procédé selon la présente invention consiste donc à transformer directement les 1-aryl-3-alkyl-1H-pyrazoline-5-one selon la formule générale (II) selon l'opération schématisée par l'étape (a) / figure 2 ou 3, telle qu'explicitée plus haut, puis à purifier le produit brut obtenu par chromatographie flash sur gel de silice, à transformer l'aldéhyde selon la formule générale (IV) correspondante en produit selon la formule générale (I) selon l'étape (d) / figure 3. Cette transformation se fait dans un milieu solvant aliphatique hydroxylé, de préférence dans le méthanol, avec dans un premier temps, pour la formation de l'imine avec le diaminomaléonitrile, une concentration molaire en substrat comprise entre 0.15 et 0.2 M, de préférence 0.18 M, avec une catalyse acide, de préférence assurée par l'acide trifluoroacétique, présent dans des proportions comprises entre 0.02 et 0.2 équivalent molaire, de préférence 0.1 équivalent molaire, puis, dans un deuxième temps pour la cyclisation oxydante et la formation du cycle imidazolyl, la dilution vers une concentration molaire en substrat comprise entre 0.01 et 0.08 M, de préférence 0.04 M et l'utilisation de 2 à 3 équivalents molaires d'hypochlorite de sodium d'une concentration allant de 2 M à 5 M, de préférence 2 équivalents molaires du produit industriel 2.3 M.

Les exemples illustrent les caractéristiques et les avantages du procédé selon la présente invention sans en limiter la portée.

### EXEMPLE 1: Préparation de la 1-(2,6-dichloro-4-trifluorométhylphényl)-3-méthyl-1H-pyrazolin-5-one (II a)

On coule à température ambiante 5.27 g d'acétoacétate d'éthyle (40.5 mmoles) sur une solution de 9.8 g de 2,6-dichloro-4-trifluorométhylphénylhydrazine (40 mmoles) dans 50 ml d'acide acétique glacial, on porte au reflux pendant 3 heures sans agitation. L'agitation est maintenue pendant le retour à la température ambiante avant l'élimination du solvant sous pression réduite. Le résidu est concrétisé dans 80 ml d'hexane pour obtenir ainsi le produit du titre avec un rendement de 85%, produit présentant les caractéristiques suivantes :
- point de fusion : 169-170°C,
- RMN ¹H (CDCl₃) : 2.02 (s, 3H) H₆ ; 3.25 (s. 2H) H₄ ; 7.5 (s, 2H) H₉ H_{9'}, RMN ¹³C : 18.0 (C₆) ; 41.5 (C₄) ; 123.0 (q, J_{C-F}=273.4 Hz, C₁₁) ; 126.6 (q, J_{C-F}=3.6 Hz, C₉, C_{9'}) ; 133.8 (q, J_{C-F}=34.4 Hz, C₁₀) ; 136.6 (C₇) ; 137.1 (C₈, C_{8'}) ; 158.6 (C₃) ; 171.9 (C₅). RMN ¹⁹F : -63.7.

Pour une meilleure compréhension des données compilées ci-dessus et dans les exemples suivants, il a été retenu une numérotation atomique qu'on peut retrouver dans la structure présentée à l'exemple 4.

### EXEMPLE 2: Préparation du 5-chloro-1-(2,6-dichloro-4-trifluorométhylphényl)-3-méthyl-1H-pyrazole-4-carboxaldéhyde (IVa), selon eq. A / fig. 2)

Dans un ballon de 500 ml équipé d'un réfrigérant et d'une ampoule de coulée, on introduit 100 ml de POCl₃ (1.09 mole). On porte la température entre 0 et 5°C pour couler lentement 2.8 ml de N, N-diméthylformamide (36.3 mmoles). Après retour vers la température ambiante en 10 à 15 minutes, on ajoute 11.3 g (36.3 mmoles) de pyrazolone (IIa). Après dissolution, l'ensemble est porté à reflux pendant 16 heures. Le mélange réactionnel est alors coulé lentement sur 1.5 litres d'eau glacée et on neutralise avec du carbonate de sodium.

Le précipité résultant est récupéré par filtration. On procède alors à une purification par chromatographie flash sur gel de silice, élution par un mélange acétate d'éthyle / pentane (5/95), pour obtenir 11.2 g du produit du titre avec un rendement de 86%, produit présentant les caractéristiques suivantes :
- point de fusion : 76°C
- RMN ¹H (CDCl₃) : 2.55 (s, 3H) H₆ ; 7.80 (s, 2H) H₉, H₉'; 10.0 (s, 1H) H₁₂. RMN ¹³C : 14.8 (C₆) ; 117.7 (C₅) ; 122.8 (q, J_{C-F}=274.2 Hz, C₁₁) ; 126.7 (q, J_{C.F}=4.0 Hz, C₉, C_{9'}) ; 135.2 (q, J_{C-F}=34.4 Hz, C₁₀) ; 136.4 (C₄) ; 136.7 (C₇) ; 137.2 (C₈, C_{8'}) ; 154.1 (C₃) ; 184.0 (C₁₂). RMN ¹⁹F: -63.7.

### EXEMPLE COMPARATIF 2 : Préparation du 5-ehloro-1-(2,6-dichloro-4-trifluorométhylphényl)-3-méthyl-1H-pyrazole-4-carboxaldéhyde (IVa), selon eqs. a₁ et a₂ / fig. 1)

Dans un ballon de 500 ml équipé d'un réfrigérant et d'une ampoule de coulée, on introduit 127 ml de POCl₃ (1.39 mole), on porte la température entre 0 et 5°C pour couler lentement 3.09 g (42.2 mmoles) de N, N-diméthylformamide. Après retour vers la température ambiante en 10 à 15 minutes, on ajoute 11.3 g (36.3 mmoles) de pyrazolone (IIa). L'ensemble est porté au reflux pendant 30 minutes, l'excès de POCl₃ éliminé sous pression réduite, le résidu coulé avec précautions dans l'eau glacée. Après neutralisation au carbonate de sodium et extraction à l'éther, le résidu est purifié par chromatographie flash sur gel de silice, élution par le mélange méthanol / chlorure de méthylène (2/98). On obtient ainsi 7.85 g de l'intermédiaire (IIIa), soit la 1-(2,6-dichloro-4-trifluorométhylphényl)-3-méthyl-4-((diméthylamino)méthylidène)-1 H-pyrazolin-5-one.

On reprend les 7.85 g de cet intermédiaire (21.4 mmoles) pour un traitement à reflux pendant 2 heures dans 240 ml de POCl₃ (4.59 moles), suivi d'une agitation à température ambiante maintenue pendant 18 heures. L'excès de POCl₃ est éliminé sous pression réduite, le résidu coulé avec précautions sur l'eau glacée. Après neutralisation au carbonate de sodium et extraction à l'éther, le résidu est purifié par chromatographie flash sur gel de silice, élution par le mélange acétate d'éthyle / pentane (5/95), pour donner 6.6 g du produit du titre, soit avec un rendement global voisin de 51%.

Caractéristiques de l'intermédiaire (IIIa) :
- point de fusion : 201°C,
- RMN ¹H (CDCl₃) : 2.20 (s, 3H) H₆ ; 3.31 (s, 3H) et 3.85 (s,3H) pour les deux radicaux méthyle (N-CH₃) ; 7.18 (s, 1H) H₁₂ ; 7.65 (s, 2H) H₉, H_{9'}. RMN ¹³C : 4.5 (C₆) ; 44.2 et 48.8 pour les deux radicaux méthyle (N-CH₃) ; 98.1 (C₄) ; 123.6 (q, J_{C-F}=273.4 Hz, C₁₁) ; 126.4 (q, J_{C-F}=3.6 Hz, C₉, C_{9'}) ; 133.8 (q, J_{C-F} =34.4 Hz, C₁₀); 137.6 (C₇) ; 138.7 (C₈, C_{8'}) ; 152.7 (C₃) ; 153.4 (C₁₂) ; 163.1 (C₅). RMN ¹⁹F : -63.7.

### EXEMPLE 3: Préparation du 4-((2-amino-1,2-dicyanoéthénylimino)méthyl)-5-chloro-1-(2,6-dichloro-4-tritluorométhylphényl)-3-méthyl-1H-pyrazole (Va), selon eq. b / fig. 2)

Dans un ballon de 500 ml équipé d'un réfrigérant, on prépare une solution de 16.1 g d'aldéhyde (IVa) (45 mmoles) et de 5 g de diaminomaléonitrile (46.3 mmoles) dans 200 ml de méthanol. A cette solution sous agitation, on ajoute 0.35 ml d'acide trifluoroacétique, soit 10% molaire. On prolonge l'agitation à température ambiante pendant 30 minutes, puis à reflux pendant 1 heure, avant de refroidir et d'éliminer les solvants sous pression réduite.

Le produit brut est concrétisé et séché. On obtient ainsi 19.7 g du produit du titre, avec un rendement voisin de 98%. Ce produit répond aux caractéristiques physiques mentionnées ci-après :
- point de fusion : 199°C,
- RMN ¹H (CDCl₃) : 2.55 (s, 3H) H₆ ; 5.30 (s, 2H) H₁₅ ; 7.80 (s, 2H) H₉, H_{9'} ; 8.40 (s, 1H) H₁₂ . RMN ¹³C : 15.8 (C₆) ; 109.2, 112.8, 114.3 (C₁₄, C₁₅ ou C_{15'}) ; 115.5 (C₅) ; 122.8 (q, J_{C-F}=273.6 Hz, C₁₁) ; 125.1 (C_{14'}) ; 126.7 (q, J_{C-F}=4.0 Hz, C₉, C_{9'}); 133.9 (C₄) ; 135.1 (q, J_{C-F}=34.4 Hz, C₁₀) ; 136.7 (C₇) ; 137.2 (C₈, C_{8'}) ; 150.2 (C₁₂) ; 152.9 (C₃) . RMN ¹⁹F : -63.7.

### EXEMPLE 4 : Préparation du 5-chloro-1-(2,6-dichloro-4-trifluorométhyluhényl)-4-(4,5-dicyano-1H-imidazol-2-yl)-3-méthyl-1H-pyrazole (Ia), selon eq. c /fig. 2, avec tBuOCl)

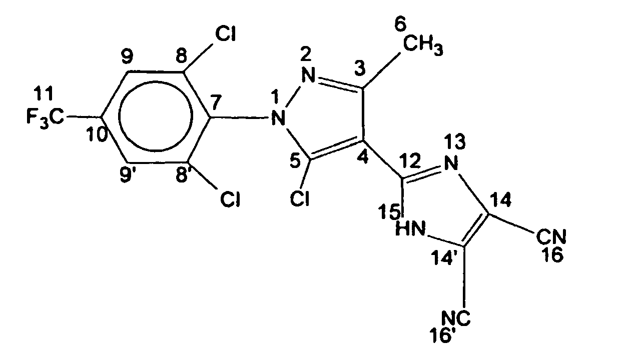

Dans un ballon de 500 ml équipé d'une ampoule de coulée et portée à une température comprise entre 0 et 5°C, on introduit 8 g d'imine (Va) (17.9 mmoles). On coule sous agitation une solution de 2.33 g d'hypochlorite de tertiobutyle (21.5 mmmoles) dans 180 ml d'acétate d'éthyle. La solution résultante est agitée à 0°C pendant 90 minutes puis à température ambiante pendant 2 heures. Le mélange réactionnel est dilué avec 80 ml d'eau puis extrait au dichlorométhane. La phase organique résultante est lavée à l'eau à trois reprises, puis séchée sur sulfate de magnésium. Après élimination du solvant sous pression réduite, le résidu est purifié par chromatographie flash sur gel de silice, élution avec le mélange méthanol / chlorure de méthylène (2/98) pour donner 6.7 g du produit du titre, avec un rendement de 83%. Ce produit répond aux caractéristiques physiques mentionnées ci-après :
- point de fusion : 98°C
- RMN ¹H (CDCl₃) : 2.68 (s, 3H) H₆ ; 7.80 (s, 2H) H₉ ; 10.80 (s, 1H) H₁₅. RMN ¹³C 15.6 (C₆) ; 108.0 (C₁₆, C_{16'}) ; 111.0 (C₁₄, C_{14'}) ; 122.6 (q, J_{C-F}=271.7 Hz, C₁₁) ; 126.8 (q, J_{C-F}=3.8 Hz, C₉, C_{9'}) ; 129.3 (C₄) ; 135.3 (q, J_{C-F}=34.6 Hz, C₁₀) ; 136.6 (C₇); 137.2 (C₈, C_{8'}) ; 144.5 (C₁₂); 153.0 (C₃) . RMN ¹⁹F : -63.7.

### EXEMPLE 5 Préparation du 5-ehloro-1-(2,6-dichloro-4-trilluorométhylphényl)-4-(4,5-dievano-1H-imidazol-2-yl)-3-méthyl-1H-pyrazole (Ia), selon eq. c / fig. 2, avec NaOCl)

Dans un ballon de 500 ml équipé d'une ampoule de coulée, on prépare une solution de 8 g d'imine (Va) (17.9 mmoles) dans 400 ml de méthanol et on la porte à 0°C. On additionne à la même température 15.7 ml (35.8 mmoles) d'une solution d'hypochlorite de sodium 2.3 M. Le mélange réactionnel est agité à température ambiante pendant 30 minutes puis coulé dans 1.3 litre d'eau. Après extractions répétées à l'acétate d'éthyle, la phase organique est lavée à trois reprises à l'eau puis séchée sur sulfate de magnésium. Après élimination du solvant sous pression réduite, le résidu est purifié par chromatographie flash sur gel de silice, élution par le mélange méthanol / chlorure de méthylène (2/98) pour donner 7 g du produit du titre, avec un rendement de 88%.

### EXEMPLE COMPARATIF 5 : Préparation du 5-chloro-1-(2,6-dichloro-4-tritluorométhylphényl -4-(4,5-dicyano-1H-imidazol-2-yl)-3-méthyl-1H-pyrazole (Ia), selon eq. c / fig. 1, avec DDQ)

Dans un ballon de 500 ml équipé d'un réfrigérant, on porte à reflux pendant 18 heures une solution de 8 g d'imine (Va) (17.9 mmoles) et de 5.9 g (26 mmoles) de 2,3-dichloro-5,6-dicyano-1,4-benzoquinone dans 140 ml d'acétonitrile. Le solvant est éliminé sous pression réduite, le résidu rouge sombre correspondant est purifié par chromatographie flash sur gel de silice, élution par le mélange méthanol / chlorure de méthylène (2/98) pour donner 4.3 g du produit du titre, avec un rendement de 54%.

### EXEMPLE COMPARATIF 5-2: Préparation du 5-chloro-1-(2,6-dichloro-4-trifluorométhylphényl)-4-(4,5-dicyano-1H-imidazol-2-yl)-3-méthyl-1H-pyrazole (Ia), selon eq. c / fig. 1, avec NCS / nicotinamide)

Dans un ballon de 250 ml on procède au mélange de 8 g d'imine (Va) (17.9 mmoles), de 2.39 g (17.9 mmoles) de N-chlorosuccinimide, de 2.44 g (20 mmoles) de nicotinamide, dans 45 ml de N, N-diméthylformamide. La solution résultante est agitée à 55-70°C pendant 1 heure, puis après retour vers la température ambiante, cette solution est coulée sur 150 ml d'eau. Après extraction au dichlorométhane, séchage et élimination du solvant sous pression réduite, le résidu correspondant est purifié par chromatographie flash sur gel de silice, élution par le mélange méthanol / chlorure de méthylène (2/98) pour donner 4.5 g du produit du titre, avec un rendement de 56%.

### EXEMPLE 6: Préparation du 5-chloro-1-(2,6-dichloro-4-trifluorométhylphényl)-4-(4,5-dicyano-1H-imidazol-2-yl)-3-méthyl-1H-pyrazole (Ia), selon eq. d / fig. 3, avec NaOCl)

Dans un ballon d'1 litre équipé d'un réfrigérant, on prépare une solution contenant 8 g (22.4 mmoles) d'aldéhyde (IVa) préparé selon l'exemple 2, 2.42 g (22.4 mmoles) de diaminomaléonitrile dans 120 ml de méthanol, on ajoute 0.18 ml d'acide trifluoroacétique, soit 10% d'équivalent molaire. La solution résultante est agitée à température ambiante pendant 30 minutes et portée au reflux pendant 1 heure. Après retour à la température ambiante, dilution avec 360 ml de méthanol et refroidissement à une température voisine de 0°C, on coule 19.6 ml (44.8 mmoles) d'une solution d'hypochlorite de sodium 2.3 M. La solution résultante est agitée pendant 30 minutes à température ambiante, avant dilution avec 1.6 litre d'eau, puis on procède à des extractions répétées à l'acétate d'éthyle. La phase organique est ensuite lavée à trois reprises à l'eau puis séchée sur sulfate de magnésium. Après élimination du solvant sous pression réduite, le résidu est purifié par chromatographie flash sur gel de silice, élution par le mélange méthanol / chlorure de méthylène (2/98) pour donner 8.2 g du produit titre, avec un rendement de 82%. Le produit du titre est obtenu ainsi à partir de la pyrazole (IIa), soit la 1-(2,6-dichloro-4-trifluorométhylphényl)-3-méthyl-1 H-pyrazolin-5-one, avec un rendement global de 70.5%.

### EXEMPLE 7: Préparation du 5-chloro-1-(2,6-dichloro-4-trifluorométhylphényl)-4-(4,5-dicyano-1H-imidazol-2-yl)-3-isopropyl-1H-pyrazole (Ib), selon eqs. a et d / fig. 3)

Dans les conditions détaillées dans l'exemple 2, à partir de la pyrazolone (IIb), soit la 1-(2,6-dichloro-4-trifluorométhylphényl)-3-isopropyl-1H-pyrazolin-5-one, on obtient l'aldéhyde correspondant (IVb), soit plus précisément le 5-chloro-1-(2,6-dichloro-4-trifluorométhylphényl)-3-isopropyl-1H-pyrazole-4-carboxaldéhyde. Le produit (IVb) est transformé dans les conditions explicitées selon l'exemple 6 pour conduire au produit du titre avec un rendement global voisin de 68%, produit présentant un point de fusion de 96-99°C.

### EXEMPLE 8: Préparation du 5-chloro-1-(2,6-dichloro-4-trifluorométhylphényl)-4-(4,5-dicyano-1H-imidazol-2-yl)-3-éthyl-1H-parazole (Ic), selon eqs. a et d / fig. 3)

Dans les conditions détaillées dans l'exemple 2, à partir de la pyrazolone (IIc), soit la 1-(2,6-dichloro-4-trifluorométhylphényl)-3-éthyl-1H-pyrazolin-5-one, on obtient l'aldéhyde correspondant (IVc), soit plus précisément le 5-chloro-1-(2,6-dichloro-4-trifluorométhylphényl)-3-éthyl-1H-pyrazole-4-carboxaldéhyde. Le produit (IVc) est transformé dans les conditions explicitées selon l'exemple 6 pour conduire au produit titre avec un rendement global voisin de 70%, produit présentant un point de fusion de 75-78°C.

### EXEMPLE 9: Préparation du 5-chloro-1-(2,6-dichloro-4-trifluorométhylphényl)-4-(4,5-dicyano-1H-imidazol-2-yl)-3-tertiobutyl-1H-pyrazole (Id), selon eqs. a et d / fig. 3)

Dans les conditions détaillées dans l'exemple 2, à partir de la pyrazolone (IId), soit la 1-(2,6-dichloro-4-trifluorométhylphényl)-4-(4,5-dicyano-1H-imidazol-2-yl)-3-tertiobutyl-1H-pyrazolin-5-one, on obtient l'aldéhyde correspondant (IVd), soit plus précisément le 5-chloro-1-(2,6-dichloro-4-trifluorométhylphényl)-3-tertiobutyl-1H-pyrazole-4-carboxaldéhyde. Le produit (IVd) est transformé dans les conditions explicitées selon l'exemple 6 pour conduire au produit du titre avec un rendement global voisin de 68%, produit présentant un point de fusion de 118-120°C.

## Revendications

1. Procédé de synthèse de dérivés 5-chloro-1-aryl-4-(4,5-dicyano-1H-imidazol-2-yl)-3-alkyl-1H-pyrazole de formule générale (1) : 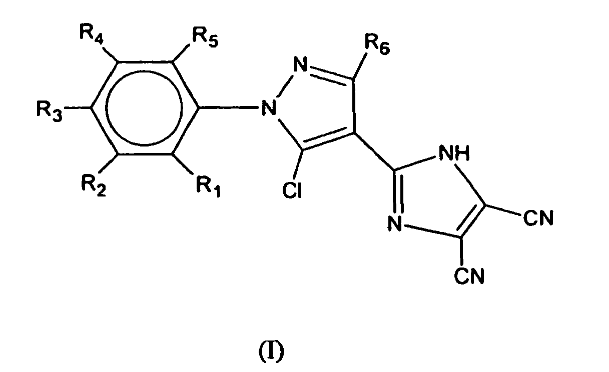 formule dans laquelle :
- R₁ à R₅, identiques ou différents, représentent un groupement choisi parmi :
* un atome d'hydrogène,
* un atome d'halogène,
* un radical répondant à la formule -(X)n-R₇ dans laquelle X représente un groupement choisi parmi l'oxygène, le soufre, un radical sulphinyle et un radical sulphonyle, n est égal à 0 ou à 1 et R₇ représente un radical alkyle linéaire ou ramifié, saturé ou insaturé éventuellement substitué par un ou plusieurs atomes d'halogène identiques ou différents, ce radical alkyle comprenant 1 à 4 atomes de carbone.
- R₆ représente un radical alkyle, linéaire ou ramifié, saturé ou insaturé, comprenant de 1 à 6 atomes de carbone, éventuellement substitués par un ou plusieurs atomes d'halogène, identiques ou différents, procédé dans lequel on utilise comme produit de départ un dérivé 1-aryl-3-alkyl-1H-pyrazoline-5-one de formule (II), ce procédé étant **caractérisé en ce que** :
(a) dans une première étape, le dérivé pyrazoline-5-one (II) est transformé en dérivé 1-aryl-3-alkyl-4-carboxaldhéhyde-5-chloro-pyrazole de formule (IV) en une étape par traitement de Vilsmeier en présence de POCL₃ et de DMF,
(b) dans une seconde étape l'aldéhyde (IV) est transformé en 1-aryl-3-alkyl-4-[(2-amino-1,2 dicyanoéthènylimino)méthyl]-5-chloro-pyrazole répondant à la formule générale (V) par condensation de l'aldéhyde (IV) avec le diaminomaléonitrile,
(c) dans une troisième étape, l'imine (V) conduit au dérivé selon la formule générale (I) par une cyclisation oxydante, qui se fait par traitement au moyen d'un hypochlorite,
suivant le schéma représenté sur la figure 2 :

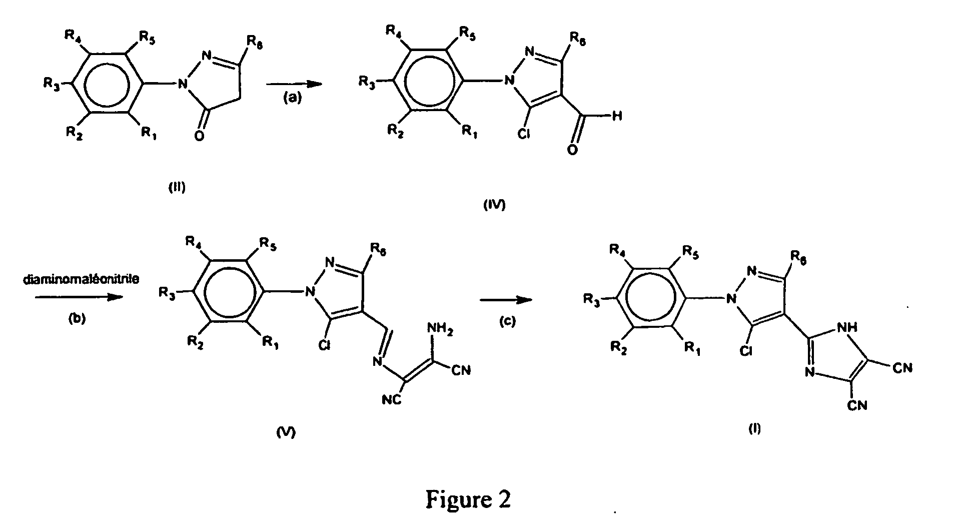

2. Procédé selon la revendication 1, **caractérisé en ce que** l'étape (a) est mise en oeuvre par traitement du composé de formule (II) dans le DMF en présence de 20 à 40 équivalents molaires de POCl₃, préférentiellement 25 à 35 équivalents molaires de POCl₃, encore plus préférentiellement 30 équivalents molaires de POCl₃.

3. Procédé selon la revendication 2, **caractérisé en ce que** le ratio (II)/DMF est compris entre 1 et 2, préférentiellement entre 1 et 1,5, encore plus préférentiellement entre 1 et 1,2.

4. Procédé selon l'une quelconque des revendications 1 à 3, **caractérisé en ce que** l'étape (b) est conduite en milieu solvant à une température comprise entre 0 et 70°C.

5. Procédé selon l'une quelconque des revendications 1 à 4, **caractérisé en ce que** l'étape (b) est conduite en milieu méthanolique avec une catalyse acide.

6. Procédé selon la revendication 5, **caractérisé en ce que** le catalyseur est l'acide trifluoroacétique.

7. Procédé selon l'une quelconque des revendications 1 à 6, **caractérisé en ce que** l'étape (c) est réalisée par traitement du composé répondant à la formule (V) par un hypochlorite choisi parmi un hypochlorite de métal alcalin ou alcalino-terreux ou un hypochlorite d'alkyle, dans un solvant aliphatique hydroxylé, à une température comprise entre -5°C et 25°C, préférentiellement entre 0°C et 5°C.

8. Procédé selon la revendication 7, **caractérisé en ce que** l'on utilise l'hypochlorite de sodium.

9. Procédé selon l'une quelconque des revendications 1 à 8, **caractérisé en ce que** l'on utilise 1 à 5 équivalents molaires d'hypochlorite par rapport au produit (V), encore plus préférentiellement 2 à 3 équivalents molaires.

10. Procédé selon l'une quelconque des revendications 1 à 9, **caractérisé en ce que** le produit de formule générale (V) est traité :
- dans le méthanol,
- à une concentration molaire de (V) allant de 0,005 M à 0,1 M, avantageusement de 0,01 M à 0,08M, encore plus préférentiellement de 0,02 M à 0,06 M,
- par un hypochlorite en quantité allant de 1 à 5 équivalents molaires, préférentiellement de 2 à 3 équivalents molaires par rapport au produit (V), cet hypochlorite étant en solution aqueuse de concentration allant de 1 à 5 M, préférentiellement de 2 à 5 M.

11. Procédé selon l'une quelconque des revendications 1 à 10, **caractérisé en ce que** les étapes (b) et (c) sont effectuées en une seule étape nommée (d), dans un même réacteur, sans isolement du produit intermédiaire (V), conformément au schéma réactionnel qui est représenté sur la figure 3 :

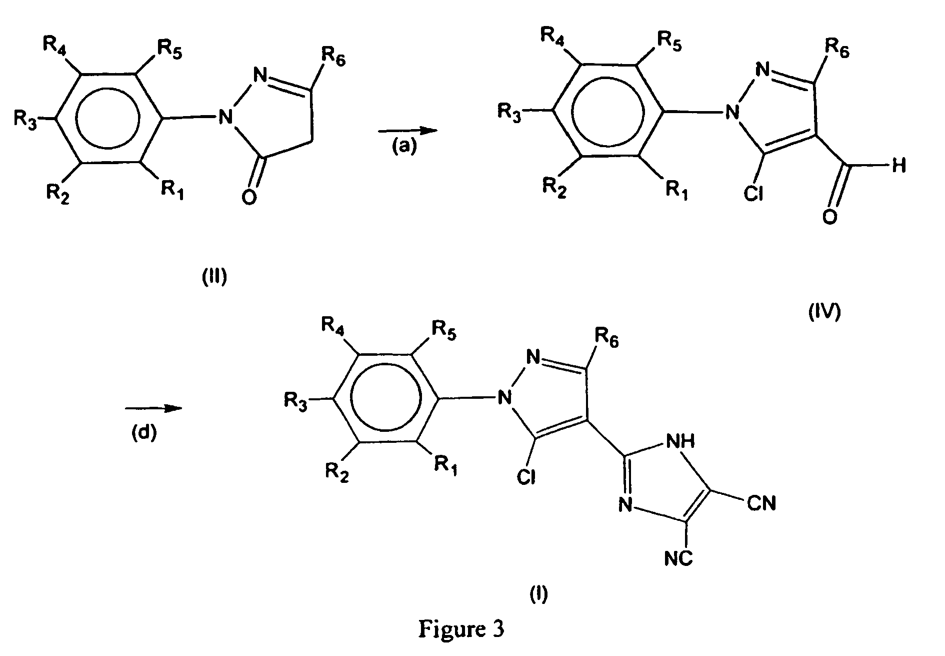

12. Procédé selon la revendication 11, **caractérisé en ce que**
(a) dans une première étape, le dérivé pyrazoline-5-one (II) est transformé en dérivé 1-aryl-3-alkyl-4-carboxaldhéhyde-5-chloro-pyrazole de formule (IV) en une étape par traitement de Vilsmeier en présence de POCL₃ et de DMF,
(d) dans une seconde étape, par traitement successif du composé de formule (IV) par le diaminomaléonitrile puis par un hypochlorite.

13. Procédé selon la revendication 12, **caractérisé en ce que** l'étape (d) se fait dans un milieu solvant aliphatique hydroxylé, avec dans un premier temps, pour la formation de l'imine avec le diaminomaléonitrile, une concentration molaire en substrat comprise entre 0.15 et 0.2 M, avec une catalyse acide, de préférence assurée par l'acide trifluoroacétique, présent dans des proportions comprises entre 0.02 et 0.2 équivalent molaire, puis, dans un deuxième temps pour la cyclisation oxydante et la formation du cycle imidazolyl, la dilution vers une concentration molaire en substrat comprise entre 0.01 et 0.08 M, et l'utilisation de 2 à 3 équivalents molaires d'hypochlorite de sodium d'une concentration allant de 2 M à 5 M.

14. Procédé selon l'une quelconque des revendications 1 à 13, **caractérisé en ce que** dans la formule (I) n=0.

15. Procédé selon l'une quelconque des revendications 1 à 14, **caractérisé en ce que** l'une ou plusieurs des conditions suivantes sont remplies :
- R₁ à R₅, identiques ou différents, représentent un groupement choisi parmi :
* un atome d'hydrogène,
* un atome d'halogène,
* un radical R₇ alkyle linéaire ou ramifié, saturé ou insaturé éventuellement substitué par un ou plusieurs atomes d'halogène identiques ou différents, ce radical alkyle comprenant 1 à 4 atomes de carbone.
- R₆ représente un radical alkyle, linéaire ou ramifié, saturé ou insaturé, comprenant de 1 à 4 atomes de carbone.

16. Procédé selon l'une quelconque des revendications 1 à 15, **caractérisé en ce que** l'une ou plusieurs des conditions suivantes sont remplies :
- R₁ à R₅, identiques ou différents, représentent un groupement choisi parmi :
* un atome d'hydrogène,
* un atome de chlore,
* un radical R₇ alkyle linéaire ou ramifié, saturé ou insaturé éventuellement substitué par un ou plusieurs atomes de fluor, ce radical alkyle comprenant 1 à 4 atomes de carbone.
- R₆ représente un radical choisi parmi le méthyle, l'éthyle, le tertiobutyle, l'isopropyle.

17. Procédé selon l'une quelconque des revendications 1 à 16, **caractérisé en ce que** le produit de formule (1) est choisi parmi :
le 5-chloro-1-(2,6-dichloro-4-trifluorométhylphényl)-4-(4,5-dicyano-1H-imidazol-2-yl)-3-méthyl-1H-pyrazole,
le 5-chloro-1-(2,6-dichloro-4-trifluorométhylphényl)-4-(4,5-dicyano-1H-imidazol-2-yl)-3-isopropyl-1H-pyrazole,
le 5-chloro-1-(2,6-dichloro-4-trifluorométhylphényl)-4-(4,5-dicyano-1H-imidazol-2-yl)-3-éthyl-1H-pyrazole,
le 5-chloro-1-(2,6-dichloro-4-trifluorométhylphényl)-4-(4,5-dicyano-1H-imidazol-2-yl)-3-tertiobutyl-1 H-pyrazole.

## Claims

1. Process for the synthesis of 5-chloro-1-aryl-4-(4,5-dicyano-1H-imidazol-2-yl)-3-alkyl-1H-pyrazole of the general formula (I): 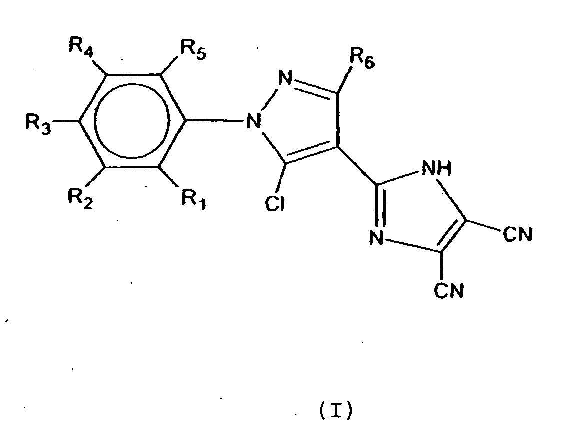 in which formula:
- R₁ to R₅, which may be identical or different, represent a group selected from:
* a hydrogen atom,
* a halogen atom,
* a radical corresponding to the formula -(X)n-R₇ in which X represents a group selected from oxygen, sulphur, a sulphinyl radical and a sulphonyl radical, n is 0 or 1 and R₇ represents a saturated or unsaturated, linear or branched alkyl radical optionally substituted by one or more identical or different halogen atoms, this alkyl radical comprising from 1 to 4 carbon atoms,
- R₆ represents a saturated or unsaturated, linear or branched alkyl radical comprising from 1 to 6 carbon atoms optionally substituted by one or more identical or different halogen atoms, in which process there is used as starting material a 1-aryl-3-alkyl-1H-pyrazoline-5-one derivative of formula (II), this process being **characterised in that**:
(a) in a first step, the pyrazoline-5-one derivative (II) is converted into a 1-aryl-3-alkyl-4-carboxaldehyde-5-chloropyrazole derivative of formula (IV) in one step by Vilsmeier treatment in the presence of POCl₃ and DMF,
(b) in a second step, the aldehyde (IV) is converted into 1-aryl-3-alkyl-4-[(2-amino-1,2-dicyanoethenylimino)methyl]-5-chloropyrazole corresponding to the general formula (V) by condensation of the aldehyde (IV) with diaminomaleonitrile,
(c) in a third step, the imine (V) leads to the derivative according to the general formula (I) by oxidising cyclisation, which is effected by treatment with a hypochlorite,
in accordance with the scheme represented in Figure 2:

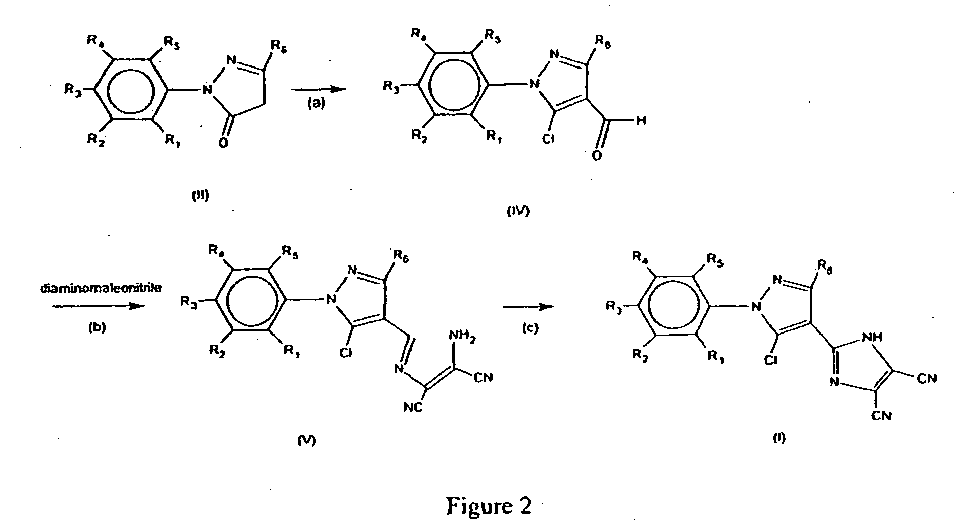

2. Process according to claim 1, **characterised in that** step (a) is implemented by treating the compound of formula (II) in DMF in the presence of from 20 to 40 molar equivalents of POCl₃, preferably from 25 to 35 molar equivalents of POCl₃, and even more preferably 30 molar equivalents of POCl₃.

3. Process according to claim 2, **characterised in that** the ratio (II)/DMF is from 1 to 2, preferably from 1 to 1.5, and even more preferably from 1 to 1.2.

4. Process according to any one of claims 1 to 3, **characterised in that** step (b) is carried out in a solvent medium at a temperature of from 0 to 70°C.

5. Process according to any one of claims 1 to 4, **characterised in that** step (b) is carried out in a methanolic medium with acid catalysis.

6. Process according to claim 5, **characterised in that** the catalyst is trifluoroacetic acid.

7. Process according to any one of claims 1 to 6, **characterised in that** step (c) is carried out by treating the compound corresponding to formula (V) with a hypochlorite selected from an alkali metal or alkaline-earth metal hypochlorite or an alkyl hypochlorite, in a hydroxylated aliphatic solvent, at a temperature of from -5°C to 25°C, preferably from 0°C to 5°C.

8. Process according to claim 7, **characterised in that** sodium hypochlorite is used.

9. Process according to any one of claims 1 to 8, **characterised in that** from 1 to 5 molar equivalents of hypochlorite are used relative to the product (V), and even more preferably from 2 to 3 molar equivalents.

10. Process according to any one of claims 1 to 9, **characterised in that** the product of the general formula (V) is treated:
- in methanol,
- at a molar concentration of (V) ranging from 0.005 M to 0.1 M, advantageously from 0.01 M to 0.08 M, and even more preferably from 0.02 M to 0.06 M,
- with a hypochlorite in a quantity ranging from 1 to 5 molar equivalents, preferably from 2 to 3 molar equivalents relative to the product (V), this hypochlorite being in an aqueous solution having a concentration ranging from 1 to 5 M, preferably from 2 to 5 M.

11. Process according to any one of claims 1 to 10, **characterised in that** steps (b) and (c) are carried out in a single step called (d), in the same reactor, without isolating the intermediate product (V), in accordance with the reaction scheme which is represented in Figure 3:

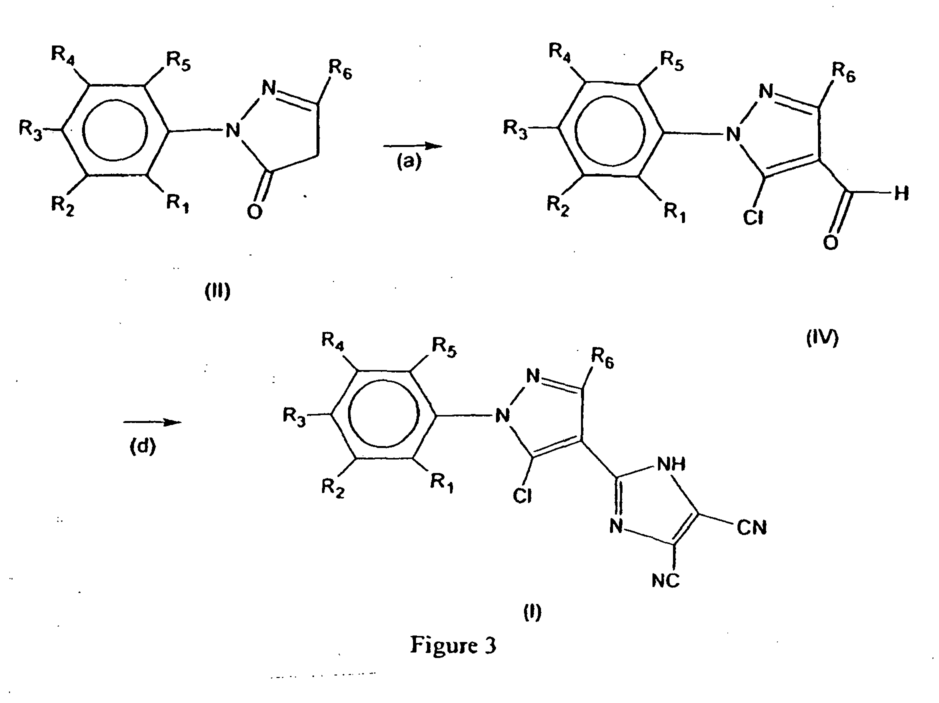

12. Process according to claim 11, **characterised in that**
(a) in a first step, the pyrazoline-5-one derivative (II) is converted into a 1-aryl-3-alkyl-4-carboxaldehyde-5-chloropyrazole derivative of formula (IV) in one step by Vilsmeier treatment in the presence of POCl₃ and DMF,
(d) in a second step, by successive treatment of the compound of formula (IV) with diaminomaleonitrile and then with a hypochlorite.

13. Process according to claim 12, **characterised in that** step (d) is carried out in a hydroxylated aliphatic solvent medium, with, in a first stage, for the formation of the imine with diaminomaleonitrile, a molar concentration of substrate of from 0.15 to 0.2 M, with acid catalysis, preferably provided by trifluoroacetic acid, which is present in proportions of from 0.02 to 0.2 molar equivalent, then, in a second stage, for the oxidising cyclisation and the formation of the imidazolyl ring, dilution to a molar concentration of substrate of from 0.01 to 0.08 M, and the use of from 2 to 3 molar equivalents of sodium hypochlorite having a concentration ranging from 2 M to 5 M.

14. Process according to any one of claims 1 to 13, **characterised in that** in formula (I) n=0.

15. Process according to any one of claims 1 to 14, **characterised in that** one or more of the following conditions are met:
- R₁ to R₅, which may be identical or different, represent a group selected from:
* a hydrogen atom,
* a halogen atom,
* a saturated or unsaturated, linear or branched alkyl radical R₇ optionally substituted by one or more identical or different halogen atoms, this alkyl radical comprising from 1 to 4 carbon atoms,
- R₆ represents a saturated or unsaturated, linear or branched alkyl radical comprising from 1 to 4 carbon atoms.

16. Process according to any one of claims 1 to 15, **characterised in that** one or more of the following conditions are met:
- R₁ to R₅, which may be identical or different, represent a group selected from:
* a hydrogen atom,
* a chlorine atom,
* a saturated or unsaturated, linear or branched alkyl radical R₇ optionally substituted by one or more fluorine atoms, this alkyl radical comprising from 1 to 4 carbon atoms,
- R₆ represents a radical selected from methyl, ethyl, tert.-butyl, isopropyl.

17. Process according to any one of claims 1 to 16, **characterised in that** the product of formula (I) is selected from:
5-chloro-1-(2,6-dichloro-4-trifluoromethylphenyl)-4-(4,5-dicyano-1H-imidazol-2-yl)-3-methyl-1H-pyrazole,
5-chloro-1-(2,6-dichloro-4-trifluoromethylphenyl)-4-(4,5-dicyano-1H-imidazol-2-yl)-3-isopropyl-1H-pyrazole,
5-chloro-1-(2,6-dichloro-4-trifluoromethylphenyl)-4-(4,5-dicyano-1H-imidazol-2-yl)-3-ethyl-1H-pyrazole,
5-chloro-1-(2,6-dichloro-4-trifluoromethylphenyl)-4-(4,5-dicyano-1H-imidazol-2-yl)-3-tert.-butyl-1H-pyrazole.

## Patentansprüche

1. Verfahren zur Synthese von 5-Chlor-1-aryl-4-(4,5-dicyano-1H-imidazol-2-yl)-3-alkyl-1H-pyrazol-Derivaten der allgemeinen Formel (I): 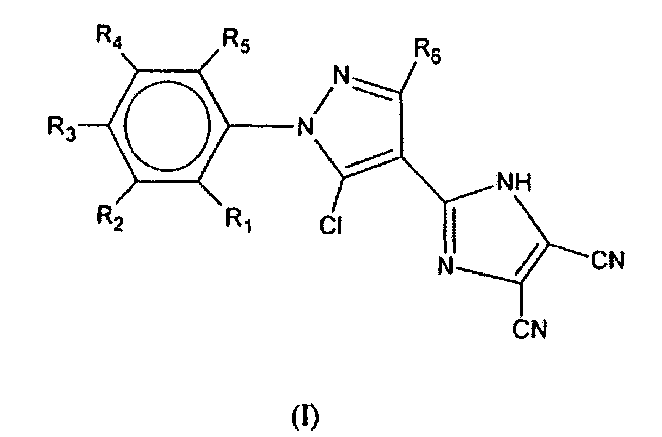 wobei in der Formel:
- R₁ bis R₅, die gleich oder verschieden sind, eine Gruppe darstellen, die ausgewählt ist aus:
* einem Wasserstoffatom,
* einem Halogenatom,
* einem Rest, der der Formel -(X)n-R₇ gehorcht, wobei X eine Gruppe darstellt, die aus Sauerstoff, Schwefel, einem Sulfinylrest und einem Sulfonylrest ausgewählt ist, n 0 oder 1 entspricht und R₇ einen linearen oder verzweigten, gesättigten oder ungesättigten Alkylrest darstellt, der gegebenenfalls mit einem oder mehreren Halogenatomen, die gleich oder verschieden sind, substituiert ist, wobei der Alkylrest 1 bis 4 Kohlenstoffatome einschließt.
- R₆ einen linearen oder verzweigten, gesättigten oder ungesättigten Alkylrest darstellt, der 1 bis 6 Kohlenstoffatome umfasst, der gegebenenfalls mit einem oder mehreren Halogenatomen, die gleich oder verschieden sind, substituiert ist,
wobei bei dem Verfahren als Ausgangsprodukt ein 1-Aryl-3-alkyl-1H-pyrazolin-5-on-Derivat der Formel (II) eingesetzt wird,
wobei dieses Verfahren **dadurch gekennzeichnet ist, dass**:
(a) in einem ersten Schritt das Pyrazolin-5-on-Derivat (II) in das 1-Aryl-3-alkyl-4-carboxaldehyd-5-chlorpyrazol-Derivat der Formel (IV) in einem Schritt durch Vilsmeier-Behandlung in Gegenwart von POCl₃ und DMF übergeführt wird,
(b) in einem zweiten Schritt der Aldehyd (IV) in 1-Aryl-3-alkyl-4-[(2-amino-1,2-dicyanoethenylimino)methyl]-5-chlorpyrazol, das der allgemeinen Formel (V) gehorcht, durch Kondensation des Aldehyds (IV) mit dem Diaminomaleinsäuredinitril übergeführt wird,
(c) in einem dritten Schritt das Imin (V) durch oxidative Cyclisierung, die durch Behandlung mit einem Hypochlorit erfolgt, das Derivat der allgemeinen Formel (I) nach dem folgenden, in Fig. 2 dargestellten Schema ergibt:

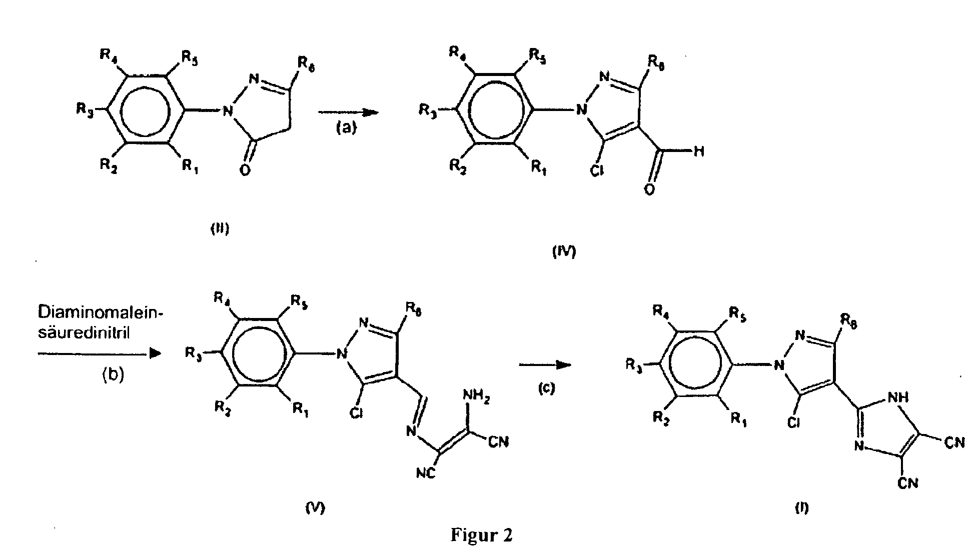

2. Verfahren nach Anspruch 1, **dadurch gekennzeichnet, dass** der Schritt (a) durch Behandlung der Verbindung der Formel (II) in DMF in Gegenwart von 20 bis 40 Moläquivalenten POCl₃, vorzugsweise 25 bis 35 Moläquivalenten POCl₃, noch bevorzugter 30 Moläquivalenten POCl₃ durchgeführt wird.

3. Verfahren nach Anspruch 2, **dadurch gekennzeichnet, dass** das Verhältnis (II)/DMF zwischen 1 und 2, vorzugsweise zwischen 1 und 1,5, noch bevorzugter zwischen 1 und 1,2 liegt.

4. Verfahren nach einem der Ansprüche 1 bis 3, **dadurch gekennzeichnet, dass** der Schritt (b) in einem Lösungsmittel-Medium bei einer Temperatur von 0 bis 70 °C durchgeführt wird.

5. Verfahren nach einem der Ansprüche 1 bis 4, **dadurch gekennzeichnet, dass** der Schritt (b) in einem methanolischen Medium unter Säure-Katalyse durchgeführt wird.

6. Verfahren nach Anspruch 5, **dadurch gekennzeichnet, dass** der Katalysator Trifluoressigsäure ist.

7. Verfahren nach einem der Ansprüche 1 bis 6, **dadurch gekennzeichnet, dass** der Schritt (c) durch Behandlung der Verbindung, die der Formel (V) gehorcht, mit einem Hypochlorit, das aus einem Alkalimetallhypochlorit oder einem Erdalkalimetallhypochlorit oder einem Alkylhypochlorit ausgewählt ist, in einem hydroxylierten aliphatischen Lösungsmittel bei einer Temperatur von -5 °C bis 25 °C, vorzugsweise von 0 °C bis 5 °C, durchgeführt wird.

8. Verfahren nach Anspruch 7, **dadurch gekennzeichnet, dass** Natriumhypochlorit verwendet wird.

9. Verfahren nach einem der Ansprüche 1 bis 8, **dadurch gekennzeichnet, dass** bezüglich Produkt (V) 1 bis 5 Moläquivalente, noch bevorzugter 2 bis 3 Moläquivalente Hypochlorit verwendet werden.

10. Verfahren nach einem der Ansprüche 1 bis 9, **dadurch gekennzeichnet, dass** das Produkt der allgemeinen Formel (V) behandelt wird:
- in Methanol,
- bei einer molaren Konzentration von (V), die von 0,005 M bis 0,1 M, zweckmäßigerweise von 0,01 M bis 0,08 M, noch bevorzugter von 0,02 M bis 0,06 M reicht,
- mit einem Hypochlorit in einer Menge, die bezüglich Produkt (V) von 1 bis 5 Moläquivalenten, vorzugsweise von 2 bis 3 Moläquivalenten reicht, wobei dieses Hypochlorit in wässriger Lösung in einer Konzentration von 1 bis 5 M, vorzugsweise von 2 bis 5 M vorliegt.

11. Verfahren nach einem der Ansprüche 1 bis 10, **dadurch gekennzeichnet, dass** die Schritte (b) und (c) in einem einzigen Schritt, der (d) genannt wird, in demselben Reaktor, ohne Isolierung des Zwischenprodukts (V), gemäß dem folgenden, in Fig. 3 dargestellten Reaktionsschema durchgeführt werden:

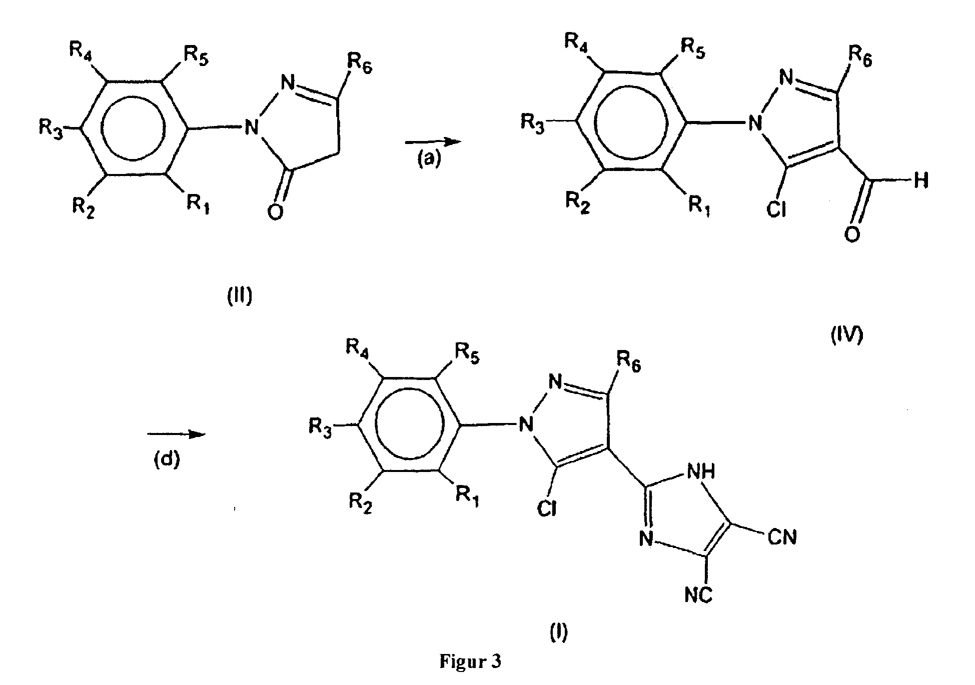

12. Verfahren nach Anspruch 11, **dadurch gekennzeichnet, dass**
(a) in einem ersten Schritt das Pyrazolin-5-on-Derivat (II) in einem Schritt durch Vilsmeier-Behandlung in Gegenwart von POCl₃ und DMF in das 1-Aryl-3-alkyl-4-carboxaldehyd-5-chlorpyrazol-Derivat der Formel (IV) übergeführt wird,
(d) in einem zweiten Schritt die Verbindung der Formel (IV) aufeinanderfolgend mit Diaminomaleinsäuredinitril und anschließend mit einem Hypochlorit behandelt wird.

13. Verfahren nach Anspruch 12, **dadurch gekennzeichnet, dass** der Schritt (d) in einem hydroxylierten aliphatischen Lösungsmittel-Medium erstens, zur Bildung des Imins mit dem Diaminomaleinsäuredinitril, mit einer molaren Konzentration an Substrat von 0,15 bis 0,2 M, unter Säure-Katalyse, die vorzugsweise durch Trifluoressigsäure, die in Anteilen von 0,02 bis 0,2 Moläquivalenten vorhanden ist, sicher gestellt wird, und anschließend zweitens, zur oxidativen Cyclisierung und Bildung des Imidazolyl-Cyclus, mit Verdünnung auf eine molare Konzentration an Substrat von 0,01 bis 0,08 M und Verwendung von 2 bis 3 Moläquivalenten Natriumhypochlorit einer Konzentration von 2 M bis 5 M erfolgt.

14. Verfahren nach einem der Ansprüche 1 bis 13, **dadurch gekennzeichnet, dass** in der Formel (I) n = 0 bedeutet.

15. Verfahren nach einem der Ansprüche 1 bis 14, **dadurch gekennzeichnet, dass** eine oder mehrere der folgenden Bedingungen erfüllt sind:
- R₁ bis R₅, die gleich oder verschieden sind, stellen eine Gruppe dar, die ausgewählt ist aus:
* einem Wasserstoffstom,
* einem Halogenatom,
* einem linearen oder verzweigten, gesättigten oder ungesättigen R₇-Alkylrest, der gegebenenfalls mit einem oder mehreren gleichen oder verschiedenen Halogenatomen substituiert ist, wobei dieser Alkylrest 1 bis 4 Kohlenstoffatome umfasst,
- R₆ schließt einen linearen oder verzweigten, gesättigten oder ungesättigten Alkylrest ein, der 1 bis 4 Kohlenstoffatome umfasst.

16. Verfahren nach einem der Ansprüche 1 bis 15, **dadurch gekennzeichnet, dass** eine oder mehrere der folgenden Bedingungen erfüllt sind:
- R₁ bis R₅, die gleich oder verschieden sind, stellen eine Gruppe dar, die ausgewählt ist aus:
* einem Wasserstoffatom,
* einem Chloratom,
* einem linearen oder verzweigten, gesättigten oder ungesättigten R₇-Alkylrest, der gegebenenfalls mit einem oder mehreren Fluoratomen substituiert ist, wobei dieser Alkylrest 1 bis 4 Kohlenstoffatome umfasst.
- R₆ stellt einen Rest dar, der aus Methyl, Ethyl, tert.-Butyl, Isopropyl ausgewählt ist.

17. Verfahren nach einem der Ansprüche 1 bis 16, **dadurch gekennzeichnet, dass** das Produkt der Formel (I) ausgewählt ist aus:
5-Chlor-1-(2,6-dichlor-4-trifluormethylphenyl)-4-(4,5-dicyano-1H-imidazol-2-yl)-3-methyl-1H-pyrazol
5-Chlor-1-(2,6-dichlor-4-trifluormethylphenyl)-4-(4,5-dicyano-1H-imidazol-2-yl)-3-isopropyl-1 H-pyrazol
5-Chlor-1-(2,6-dichlor-4-trifluormethylphenyl)-4-(4,5-dicyano-1H-imidazol-2-yl)-3-ethyl-1H-pyrazol
5-Chlor-1-(2,6-dichlor-4-trifluormethylphenyl)-4-(4,5-dicyano-1H-imidazol-2-yl)-3-tertbutyl-1 H-pyrazol.
